# EUROPEAN PATENT APPLICATION

(11) **EP 3 878 378 A1**
(43) Date of publication of application: **15.09.2021**
(21) Application number: 21161771.7
(22) Date of filing: 10.03.2021
(51) Int. Cl.: A61B 17/072

(54) **SURGICAL STAPLER WITH DISPLAY**

(30) Priority: 11.03.2020 US 202062987997 P; 23.02.2021 US 202117182335
(71) Applicant: Covidien LP, Mansfield, MA 02048 (US)
(72) Inventor: KURUVATTI, Pradeep, 500032 Hyderabad (IN); KATREVPALLI, Radhika, 500054 Hyderabad (IN); SALLA, Sowmya, 505527 Karimnagar (IN)
(74) Representative: Maschio, Antonio

(57) **Abstract**

A surgical stapler includes a handle assembly and an end effector connected by an elongate body. The end effector includes a cartridge assembly having staples, an anvil to assist forming staples, and a sensor indicating force being applied to the tissue to the stapled. The end effector also possesses a display including visual outputs that indicate to the end-user when a sufficient amount of force is being applied to the tissue and the surgical stapler should be fired.

## Description

### CROSS-REFERENCE TO RELATED APPLICATIONS

This application claims the benefit of and priority to U.S. Provisional Patent Application Serial No. 62/987,997 filed March 11, 2020, the entire disclosure of which is incorporated by reference herein.

### TECHNICAL FIELD

The disclosure relates to a surgical stapler for implanting mechanical surgical fasteners into the tissue of a patient and, in particular, to a surgical stapler which provides feedback to the user in response to one or more sensed feedback signals.

### BACKGROUND

Surgical staplers have sensors which measure various tissue properties, such as the force being exerted on tissue by the stapler. Such devices frequently have displays, indicators, etc., to provide feedback to the clinician using the device.

Concentrating on multiple devices at the same time can be challenging. For example, with some staplers, a surgeon needs to pay attention to a laparoscopic television to observe the surgical site and the surgeon also needs to observe the display associated with the stapler to identify force levels on the tissue while clamping and cutting. This can require frequent neck movements to focus on both the laparoscopic display and the stapler display.

Ergonomic issues can thus arise for end users of these devices, including surgeons. Such ergonomic issues include increased musculoskeletal complaints of neck and back pain and/or discomfort, which may arise due to dynamic posture maintenance required to watch both laparoscopic television and surgical stapler displays.

### SUMMARY

According to aspects of the present disclosure, a surgical stapler includes a handle assembly, an end effector coupled to the handle assembly, a sensor, and a display on the end effector capable of indicating force exerted on tissue. The end effector includes a first jaw member including a cartridge assembly and a second jaw member including an anvil portion. The first and second jaw members are moveable relative to one another between an open position and a clamped position. The sensor is configured to measure a clamping force exerted on tissue by the first and second jaw members. The display provides a visual indication of acceptable forces in the tissue prior to firing the surgical stapler.

In aspects of the disclosure, a surgical stapler includes a handle assembly, an end effector including a cartridge assembly and an anvil, and an elongate body connecting the handle assembly and the end effector. The surgical stapler also includes a sensor for measuring force applied to tissue by the cartridge assembly and the anvil, and a display on the end effector which provides feedback from the sensor to a user.

In other aspects of the disclosure, a surgical stapler, includes a handle assembly, an end effector including a cartridge assembly and an anvil, and an elongate body connecting the handle assembly and the end effector. The surgical stapler also includes a sensor for measuring force applied to tissue by the cartridge assembly and the anvil, and a display on the end effector which provides feedback from the sensor to a user, the display including a plurality of visual outputs.

### BRIEF DESCRIPTION OF THE DRAWINGS

Embodiments of the disclosure are described herein with reference to the accompanying drawings, wherein:

FIG. 1 is a side perspective view illustrating a surgical stapler including a handle assembly, a shaft portion, and an end effector;

FIG. 2 is a perspective view, with parts separated, illustrating the end effector of the surgical stapler of FIG. 1; and

FIG. 3 illustrates the surgical stapler of FIG. 1 in use.

### DETAILED DESCRIPTION

The disclosed surgical staplers are described in detail with reference to the drawings, in which like reference numerals designate identical or corresponding elements in each of the several views. As used herein the term "distal" refers to that portion of the surgical stapler, or component thereof, farther from the user, while the term "proximal" refers to that portion of the surgical stapler, or component thereof, closer to the user.

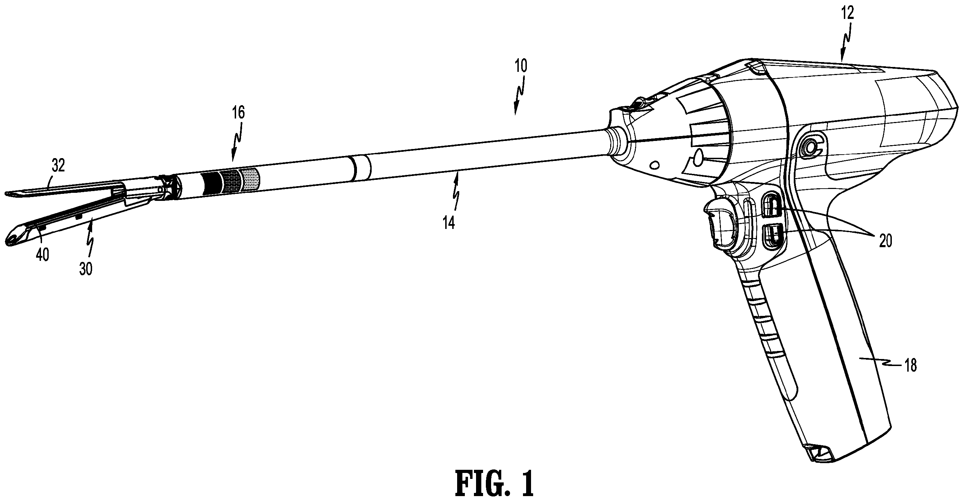

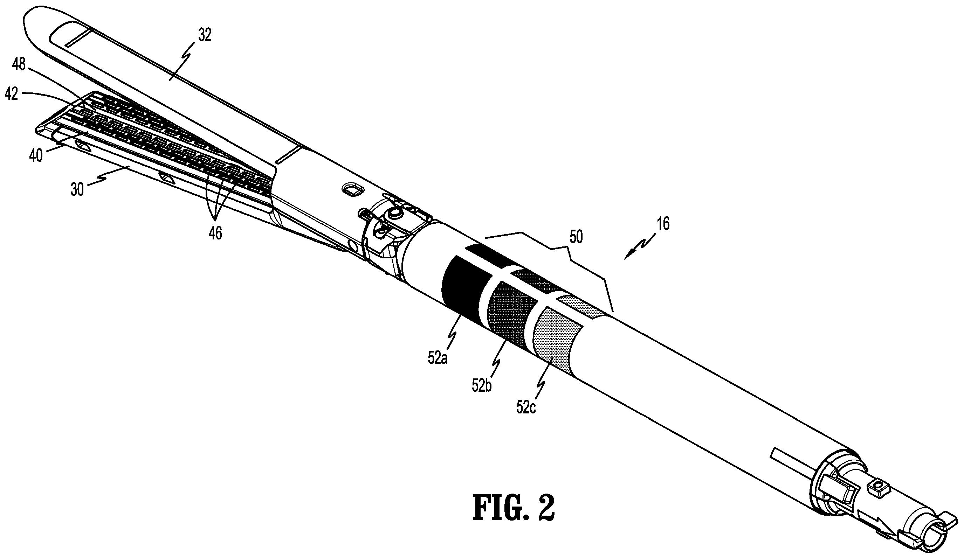

With reference to FIGS. 1 and 2, a surgical stapler in accordance with an aspect of the disclosure is shown generally as surgical stapler 10 that includes a handle assembly 12, an elongate body or adapter 14, and an end effector 16. In the illustrated aspect, the handle assembly 12 is powered and includes a stationary handgrip 18 and actuation buttons 20. The actuation buttons 20 are operable to actuate various functions of the end effector 16 via the adapter 14, including approximation, stapling, and dissection. In aspects of the disclosure, the handle assembly 12 supports batteries (not shown) that provide energy to the handle assembly 12 to operate the surgical stapler 10. Although the surgical stapler 10 is illustrated as a powered surgical stapler, it is envisioned that the advantages of this disclosure are suitable for use with manually powered surgical staplers as well as robotically controlled surgical staplers.

According to the disclosure, end effector 16 includes a cartridge assembly 30 having one or more surgical fasteners and a second opposing jaw member including an anvil 32 for forming the staples. As is known in the art, the cartridge assembly 30 and the anvil 32 are coupled together such that the end effector 16 can pivot between an open position and a clamped position. The cartridge assembly 30 includes an exemplary aspect of the disclosed staple cartridge shown generally as staple cartridge 40.

In certain aspects of the disclosure, the staples are housed in cartridge assembly 30 to apply linear rows of staples to body tissue either in simultaneous or sequential manner. Either one or both of the anvil 32 and the cartridge assembly 30 are movable in relation to one another between an open position in which the anvil 32 is spaced from cartridge assembly 30 and an approximated or clamped position in which the anvil 32 is in juxtaposed alignment with cartridge assembly 30.

FIG. 2 illustrates the end effector 16 and the staple cartridge 40 which includes a cartridge body 42 and a plurality of staples (not shown). The cartridge body 42 defines a plurality of staple pockets 46 and a central knife slot 48 that extends along a midline of the cartridge body 42. As known in the art, the central knife slot 48 facilitates translation of a knife bar (not shown) through the end effector 16 to eject staples supported within a staple cartridge and cut tissue clamped between the cartridge assembly 30 and anvil 32. U.S. Patent No. 5,865,361 discloses a manually powered surgical stapler including a knife bar that is movable through the tool assembly to eject staples from a staple cartridge and to cut tissue clamped between an anvil and a cartridge assembly of the surgical stapler. Although the cartridge body 42 is illustrated as being linear, it is envisioned that the cartridge body 42 may have a non-linear configuration or curved along its longitudinal axis.

It is envisioned that the staple pockets 46 in the cartridge body 42 need not be arranged in rows as illustrated above but rather may be arranged in a variety of different patterns in the cartridge body.

The end effector 16 also possesses a sensor (not shown) which senses the force applied to tissue by the cartridge assembly 30 and the anvil 32 while clamping and firing. The sensor is in communication (e.g., wireless or wired) with display 50.

Display 50 includes visual outputs 52a, 52b, and 52c to relay feedback to the user. The visual outputs 52a, 52b, and 52c may include corresponding indicators of various shapes, sizes and/or colors. In embodiments, the visual outputs 52a, 52b, and 52c may include one or more colored visible lights or light emitting diodes ("LED"). The visual outputs 52a, 52b, and 52c are disposed on the end effector 16 for better visibility of feedback from the sensor.

The visual outputs 52a, 52b, and 52c may display light of a certain color and/or in a certain combination to illustrate to the user the force being applied to tissue. For example, the visual outputs 52a, 52b, and 52c can include a first light (e.g., red) 52a, a second light (e.g., yellow) 52b, and a third light (e.g., green) 52c. The lights indicate an acceptable or non-acceptable level of force being applied to tissue, with green 52c indicating a sufficient level of force for firing the surgical stapler, yellow 52b indicating an acceptable level of force for firing the surgical stapler, but beginning to indicate an undesired level of force, and red 52a indicating an undesirable level of force, which should indicate to the user that the surgical stapler should not be fired, but, rather, the tissue should be re-engaged and a staple deployed when an acceptable level of force is indicated by a green visual output 52c or a yellow visual output 52b.

The disclosed surgical staplers display data related to tissue forces on the end effector 16, so that the surgeon can view forces on the tissue on the surgical stapler 10 particularly on the end effector 16 itself, either directly or by viewing through a laparoscopic display, without moving the surgeon's focus out of the surgical site.

This solution is more ergonomic for the end user, such as a surgeon, as the end user only needs to focus on the end effector of the surgical stapler, either directly or by viewing through a laparoscopic display, to view the surgical site as well as the device warnings and indications.

Any of the components described herein may be fabricated from either metals, plastics, resins, composites or the like taking into consideration strength, durability, wearability, weight, resistance to corrosion, ease of manufacturing, cost of manufacturing, and the like.

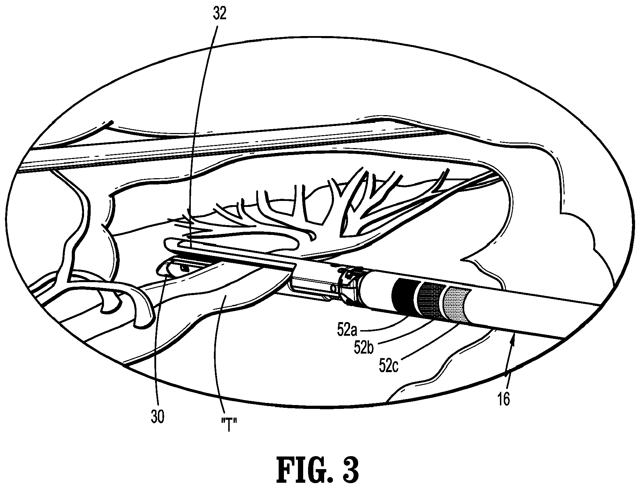

With reference to FIG. 3, operation of the surgical stapler 10 will now be described. The trigger (not shown) is depressed so that tissue "T" is encompassed between the anvil 32 and the cartridge assembly 30 of the end effector 16 and the anvil 32 and cartridge assembly 30 close about the tissue "T". The level of force applied to the tissue "T" is indicated on visual outputs 52a, 52b and 52c; once the surgeon observes that an acceptable level of force is being applied to the tissue "T", either by lighting of the green visual output 52c, or the yellow visual output 52b, the surgeon may proceed to fire the surgical stapler 10. If the red visual output 52a is observed, the surgeon should open the jaws of the surgical stapler 10 so that the anvil 32 and the cartridge assembly 30 are no longer engaged with the tissue "T" and then re-deploy the anvil 32 and cartridge assembly 30 so that an appropriate level of force is being applied to the tissue "T" as indicated by the green visual output 52c, or the yellow visual output 52b.

It will be understood that various modifications may be made to the embodiments of the presently disclosed surgical staplers. Therefore, the above description should not be construed as limiting, but merely as exemplifications of embodiments. Those skilled in the art will envision other modifications within the scope and spirit of the disclosure. For example, any and all features of one described embodiment may be suitably incorporated into another embodiment.

The invention may be described by reference to the following numbered paragraphs:
1. A surgical stapler, comprising: a handle assembly; an end effector including a cartridge assembly and an anvil; an elongate body connecting the handle assembly and the end effector; a sensor for measuring force applied to tissue by the cartridge assembly and the anvil; and a display on the end effector which provides feedback from the sensor to a user.
2. The surgical stapler according to paragraph 1, wherein the display includes a plurality of visual outputs.
3. The surgical stapler according to paragraph 2, wherein the visual outputs are of various shapes.
4. The surgical stapler according to paragraph 2, wherein the visual outputs are of various sizes.
5. The surgical stapler according to paragraph 2, wherein the visual outputs are of various colors.
6. The surgical stapler according to paragraph 2, wherein the visual outputs are a red color, a yellow color, and a green color.
7. A surgical stapler, comprising: a handle assembly; an end effector including a cartridge assembly and an anvil; an elongate body connecting the handle assembly and the end effector; a sensor for measuring force applied to tissue by the cartridge assembly and the anvil; and a display on the end effector which provides feedback from the sensor to a user, the display including a plurality of visual outputs.
8. The surgical stapler according to paragraph 7, wherein the visual outputs are of various shapes.
9. The surgical stapler according to paragraph 7, wherein the visual outputs are of various sizes.
10. The surgical stapler according to paragraph 7, wherein the visual outputs are of various colors.
11. The surgical stapler according to paragraph 7, wherein the visual outputs are a red color, a yellow color, and a green color.

## Claims

1. A surgical stapler, comprising:
a handle assembly;
an end effector including a cartridge assembly and an anvil;
an elongate body connecting the handle assembly and the end effector;
a sensor for measuring force applied to tissue by the cartridge assembly and the anvil; and
a display on the end effector which provides feedback from the sensor to a user.

2. The surgical stapler according to claim 1, wherein the display includes a plurality of visual outputs.

3. The surgical stapler according to claim 2, wherein the visual outputs are of various shapes.

4. The surgical stapler according to claim 2 or claim 3, wherein the visual outputs are of various sizes.

5. The surgical stapler according to any of claims 2 to 4, wherein the visual outputs are of various colors.

6. The surgical stapler according to claim 5, wherein the visual outputs are a red color, a yellow color, and a green color.

7. A surgical stapler, comprising:
a handle assembly;
an end effector including a cartridge assembly and an anvil;
an elongate body connecting the handle assembly and the end effector;
a sensor for measuring force applied to tissue by the cartridge assembly and the anvil; and
a display on the end effector which provides feedback from the sensor to a user, the display including a plurality of visual outputs.

8. The surgical stapler according to claim 7, wherein the visual outputs are of various shapes.

9. The surgical stapler according to claim 7 or claim 8, wherein the visual outputs are of various sizes.

10. The surgical stapler according to any of claims 7 to 9, wherein the visual outputs are of various colors.

11. The surgical stapler according to claim 10, wherein the visual outputs are a red color, a yellow color, and a green color.
